# EUROPÄISCHE PATENTSCHRIFT

(11) **EP 3 755 815 B1**
(45) Veröffentlichungstag und Bekanntmachung des Hinweises auf die Patenterteilung: **12.07.2023**
(21) Anmeldenummer: 19714312.6
(22) Anmeldetag: 04.02.2019
(51) Int. Cl.: C12Q 1/6883

(54) **BIOMARKER ZUR FESTSTELLUNG EINER MÖGLICHEN UNVERTRÄGLICHKEIT GEGENÜBER METALLIMPLANTATEN**
BIOMARKER FOR DETECTING A POSSIBLE INCOMPATABILITY WITH RESPECT TO METAL IMPLANTS
BIOMARQUEUR POUR IDENTIFIER UNE POSSIBLE INTOLÉRANCE AUX IMPLANTS MÉTALLIQUES

(30) Priorität: 23.02.2018 DE 102018104133
(43) Veröffentlichungstag der Anmeldung: 30.12.2020
(73) Patentinhaber: Klinikum der Universität München, 81377 München (DE)
(72) Erfinder: SUMMER, Burkhard, 81377 München (DE); THOMAS, Peter, 80337 München (DE); USBECK, Sylvia, 04229 Leipzig (DE); WITTMANN, Diana, 80337 München (DE)
(74) Vertreter: Weidner Stern Jeschke
(86) Internationale Anmeldenummer: PCT/DE2019/200006
(87) Internationale Veröffentlichungsnummer: WO 2019/161853

(56) Entgegenhaltungen:
- WO-A2-2009/067473
- JANSSON HENRIK ET AL: "Clinical consequences of IL-1 genotype on early implant failures in patients under periodontal maintenance.", CLINICAL IMPLANT DENTISTRY AND RELATED RESEARCH 2005, Bd. 7, Nr. 1, 2005, Seiten 51-59, XP002790891, ISSN: 1523-0899
- MALIK M H A ET AL: "Genetic susceptibility to total hip arthroplasty failure: a preliminary study on the influence of matrix metalloproteinase 1, interleukin 6 polymorphisms and vitamin D receptor", ANNALS OF THE RHEUMATIC DISEASES, Bd. 66, Nr. 8, August 2007 (2007-08), Seiten 1116-1120, XP002790892,
- MONTES CLAUDIA CRISTINA ET AL: "Analysis of the association of IL1B (C+3954T) and IL1RN (intron 2) polymorphisms with dental implant loss in a Brazilian population.", CLINICAL ORAL IMPLANTS RESEARCH FEB 2009, Bd. 20, Nr. 2, 21. Januar 2009 (2009-01-21), Seiten 208-217, XP002790893, ISSN: 1600-0501
- SAMPAIO FERNANDES MARGARIDA ET AL: "The role ofIL-1gene polymorphisms (IL1A,IL1B, andIL1RN) as a risk factor in unsuccessful implants retaining overdentures", JOURNAL OF PROSTHODONTIC RESEARCH, Bd. 61, Nr. 4, 17. Februar 2017 (2017-02-17), Seiten 439-449, XP085247377, ISSN: 1883-1958, DOI: 10.1016/J.JPOR.2017.01.004
- PÉREZ-RAMÍREZ CRISTINA ET AL: "Interleukins as new prognostic genetic biomarkers in non-small cell lung cancer", SURGICAL ONCOLOGY, BLACKWELL SCIENTIFIC PUBL., OXFORD, GB, Bd. 26, Nr. 3, 22. Mai 2017 (2017-05-22), Seiten 278-285, XP085181876, ISSN: 0960-7404, DOI: 10.1016/J.SURONC.2017.05.004
- RAMÍREZ-PÉREZ S ET AL: "Association of 86 bp variable number of tandem repeat (VNTR) polymorphism of interleukin-1 receptor antagonist (IL1RN) with susceptibility and clinical activity in rheumatoid arthritis", CLINICAL RHEUMATOLOGY, ACTA MEDICA BELGICA, BRUXELLES, BE, Bd. 36, Nr. 6, 25. März 2017 (2017-03-25), Seiten 1247-1252, XP036249435, ISSN: 0770-3198, DOI: 10.1007/S10067-017-3610-0 [gefunden am 2017-03-25] in der Anmeldung erwähnt

## Beschreibung

Die Erfindung betrifft ein Verfahren zur Prognostizierung eines erhöhten Risikos für eine erhöhte Unverträglichkeitssneigung gegenüber Metallimplantaten.

Implantate spielen beispielsweise im orthopädischen und zahnmedizinischen Bereich zunehmend eine wichtige Rolle. Derartige Implantate sind häufig aus Metall, beispielsweise Reintitan, bzw. Metalllegierungen. Damit einhergehend steigt allerdings die Anzahl von Implantat-Unverträglichkeitsreaktionen [1-4]. Beispielsweise können selbst geringfügige Mengen Verunreinigungen oder Fremdmaterialien im Metall, z.B. Nickel, zu einer Entzündungsreaktion oder allergischen Reaktion führen. Auch Abriebpartikel, die von Makrophagen und Monozyten aufgenommen werden, oder Korrosionsprozesse können Entzündungsreaktionen auslösen. Es werden bei Endoprothesen aber auch Komplikationen festgestellt, die nicht auf bekannte Ursachen wie Korrosions- oder Abriebprodukte oder Infektionen zurückgeführt werden können. So werden bislang rätselhafte überschießende Entzündungsreaktionen festgestellt, die beispielsweise mit Schwellungen, rezidivierenden Gelenkergüssen, Schmerzen, Bewegungseinschränkungen, einer Lockerung oder dem Versagen eines Implantats einhergehen können.

In JACOBI-GRESSER, E. u.a. (Genetic andimmunological markers predicttitanium implant failure: aretrospective study. Int. J. OralMaxillofac. Surg. (2013) 42 (4) 537-543) werden diagnostische Marker ausgewertet, um das Versagen von Titanimplantaten vorherzusagen. Das Implantatergebnis wurde bei 109 Probanden mit Titanimplantatchirurgie, IL1A -889 C/T (rs1800587), IL1B +3954 C/T (rs1143634), II,1RN +2018 T/C (rs419598) und TNFA -308 G/A (rs1800629) Genotypisierung, in-vitro-IL-1β/TNF-α-Freigabetests und Lymphozytentransformationstests während der Behandlung bewertet.

In RAMIREZ-PEREZ, S. u.a. (Association of 86 bp variable number of tandem repeat (VNTR) polymorphism of interleukin-1 receptor antagonist (II,1RN) with susceptibility and clinical activity in rheumatoid arthritis. Clin. Rheumatol. (2017) 36 (6) 1247-1252) wird berichtet, dass mehrere Studien eine variable Anzahl von Tandem-Wiederholungen (VNTR) 86 bp (rs2234663) im Intron 2 des IL1RN-Gens mit Rheumatoidem Arthritis-(RA)-Risiko haben. In der vorliegenden Studie sollte die Häufigkeit dieses Polymorphismus bei Patienten mit RA und Kontrollpersonen (CS) und seine Assoziation mit RA in einer westlichen mexikanischen Bevölkerung bestimmt werden. Es wurde eine analytische Querschnittsstudie durchgeführt, in die 350 Patienten mit RA und 307 CS einbezogen wurden. Die Identifizierung des II,1RN VNTR Polymorphismus wurde mittels Polymerase-Kettenreaktion (PCR) durchgeführt und Genotypen wurden mit klinischen Variablen (DAS28 und CRP) assoziiert.

In JACOBI-GRESSER, E. (Prognose der Einheilquote von Titanimplantaten anhand von Laborparametern - eine retrospektive Studie, umweltmedizin-gesellschaft 26 (2/2013) 98-103) bewerten die Autoren in ihrer Studie diagnostische Marker zur Vorhersage von Titanimplantatversagen. Das Implantatergebnis wurde rückblickend bei 109 Probanden bewertet, die sich einer Titanimplantatoperation unterzogen hatten, IL1A -889 C/T (rs1800587), IL1B +3954 C/T (rs1143634), ILIRNN +2018 T/C (rs419598) und TNFA -308 G/A (rsl 800629) Genotypisierung, in vitro IL-1 β/TNF-a Freisetzungstests und LymphozytenTransformationstests während der Behandlung.TNF-a und IL-1 β Freisetzung auf Titan-Stimulation waren signifikant höher bei den Patienten mit Implantatverlust (TNF-a: 256,89pg/ml vs. 81,4pg/ml); p<0.0001; IL-1 β: 159,96pg/ml vs. 54,01 pg/ml; p<0,0001).

In LIU, Y.H. u.a. (Association of interleukin-1beta (IL1B) polymorphisms with Graves' ophthalmopathy in Taiwan Chinese patients. Invest. Ophthalmol. Vis. Sci. (2010) 51 (12) 6238-46) wird untersucht, ob Variationen im IL1B-Gen mit der Graves'schen Ophthalmopathie (GO) bei Patienten mit Morbus Basedow (GD) assoziiert werden können. In den untersuchten II,1B-SNPs war das C-Allel von rs1143634 mit GD assoziiert, während der T/T-Genotyp der SNPs rs1143634 und rs16944 weniger mit der Krankheit assoziiert waren. Der A/A-Genotyp der SNPs rs3917368 und rs1143643, die die stärkste Interaktion hatten, kann das Risiko von GO erhöhen (P = 0,024 bzw. P = 0,017). Mehrere GD-Empfindlichkeits- und Unempfindlichkeitshaplotypen ILIB wurden identifiziert, und der Ht4-GCGCCTCC-Haplotyp, der aus acht SNPs besteht und mit niedrig zirkulierenden IL1β-Werten assoziiert ist, kann vor der Entwicklung von GO schützen (P = 0,025). Die Daten für IL1B-Polymorphismen und die Assoziation von GD und GO mit dem Plasma IL1β zeigen, dass IL1B-Polymorphismen mit der Entwicklung von GD und GO assoziiert werden können. Weiterhin beschreiben J. Henrik et al., Clinical implant Dentistry and related research, Bd7, Nr.1, 2005, 61-59; Malik M. et al., Annals of rheumatic diseases, Bd.66, Nr.8, 2007, 1116-1120; Montes C. et al., Clin. oral implants research, Bd.20, Nr.2, 2009, 208-2017 und Sampaio F. M. et al., J. Prosthodontic research, Bd.61, Nr.4, 439-449, Polymorphismen assoziierte Metallimplantat-Komplikationen.

Aufgabe der vorliegenden Erfindung ist es, die Prognostizierung von möglichen Komplikationen beim Einsatz von Metallimplantaten zu verbessern.

Zur Lösung der Aufgabe stellt die Erfindung ein Verfahren zur Prognostizierung eines erhöhten Risikos für eine erhöhte Unverträglichkeitssneigung gegenüber Metallimplantaten bei einer Testperson bereit, wobei in einer peripheren Blutprobe mit genetischem Material der Testperson:
a) eine Einzelnukleotidvariation rs1143627 
AGCCTCCTACTTCTGCTTTTGAAAGC[C/T]ATAAAAACAGCGAGGGAGAACTGG,
SEQ ID NO: 1,
 im Gen kodierend für Interleukin 1 beta, untersucht und festgestellt wird, ob ein C an der Position der Einzelnukleotidvariation vorhanden ist und/oder
b) ein VNTR-Polymorphismus rs2234663 mit der Wiederholungseinheit 
ATCCTGGGGAAAGTGAGGGAAATATGGACATCACATGGAACAACATCCAGGAGAC
TCAGGCCTCTAGGAGTAACTGGGTAGTGTGC, SEQ ID NO: 2,
 im Gen kodierend für den II,1-Rezeptorantagonisten, II,1-RN, untersucht und festgestellt wird, ob die Wiederholungseinheit fünfmal vorhanden ist.

Es hat sich überraschend herausgestellt, dass die Polymorphismen rs1143627 und rs2234663 als Biomarker zur Ermittlung eines möglicherweise erhöhten Risikos geeignet sind, dass eine Person, die ein Metallimplantat erhalten soll oder bereits erhalten hat, eine Unverträglichkeit mit dem Implantat entwickelt. Dabei kann von einem erhöhten Risiko ausgegangen werden, wenn bei der Einzelnukleotidvariation rs1143627 C und nicht T an der Position der Einzelnukleotidvariation steht, und/oder wenn bei dem VNTR-Polymorphismus rs2234663 die Wiederholungseinheit ("repeat") fünfmal tandemartig hintereinander vorhanden ist. Die Unverträglichkeit kann sich insbesondere in einer erhöhten Entzündungsneigung manifestieren, die zu Implantatlockerung (mit Osteolyse), Schmerzen, Ergüssen, Funktionsverschlechterung (z.B. reduzierte Beweglichkeit von Gelenkprothesen) führt.

Ohne an eine bestimmte Theorie gebunden sein zu wollen, wird angenommen, dass die Biomarker ein genetisch bedingtes Ungleichgewicht der Entzündungsregulation anzeigen. Dies führt zu Unverträglichkeit in Antwort auf den "Fremdkörper" Implantat. Das Immunsystem des Menschen reagiert individuell unterschiedlich stark auf ein und denselben Entzündungsreiz (z.B. Bakterien, Implantatmaterialien), wobei der Verlauf der Immunantwort durch das Verhältnis der pro- und antientzündlichen Botenstoffe (z.G. Zytokine) zueinander bestimmt wird, die eine bedeutende Rolle im Abwehrmechanismus spielen. Das Zytokin II,-1 gehört zu den wichtigsten entzündungsfördernden Botenstoffen. Eine beispielsweise durch einen genetischen Polymorphismus bedingte übermäßig hohe Freisetzung dieses Botenstoffs kann zu einer Überreaktion des Immunsystems führen. Die entzündungsfördernde Wirkung von IL-1 wird normalerweise durch den Antagonisten IL-RN herabreguliert. Wenn allerdings auch hier ein wirkungsmindernder Polymorphismus vorliegt, mangelt es an der zum Entzündungsbotenstoff IL-1 gegensteuernden Entzündungshemmung, wodurch es in der Folge zu einer übermäßigen Entzündungsreaktion kommen kann. Dies kann zumindest teilweise Fälle erklären, bei denen ohne erkennbare Ursache eine starke entzündliche Reaktion auf ein Implantat erfolgt.

Das erfindungsgemäße Verfahren kann beispielsweise dazu dienen, bei einer Person, die ein Implantat benötigt, vor der Implantation festzustellen, ob hier bei Einsatz eines Metallimplantates mit höherer Wahrscheinlichkeit mit Komplikationen, insbesondere übermäßigen Entzündungsreaktionen, gerechnet werden muss. In einem solchen Fall kann beispielsweise auf ein anderes Implantatmaterial, z.B. Keramik, ausgewichen werden. Die Erfindung kann vorteilhaft nicht nur beispielsweise auf dem Gebiet der Orthopädie oder Endoprothetik, sondern auch bei Zahnimplaten sein.

Unter dem Begriff "erhöhte Unverträglichkeitsneigung gegenüber Metallimplantaten" wird eine gegenüber der durchschnittlichen Gesamtbevölkerung erhöhte Wahrscheinlichkeit verstanden, eine Unverträglichkeitsreaktion, insbesondere eine Entzündungsreaktion, gegenüber einem Metallimplantat verstanden.

Unter einem "Implantat" oder einer "Endoprothese" wird ein im Körper eingepflanztes künstliches Material verstanden, das permanent oder zumindest für einen längeren Zeitraum dort verbleiben soll. Beispiele für Implantate sind Hüftgelenks-, Kniegelenks-, Schultergelenks- oder Zahnimplantate. Unter den Begriff fallen aber auch Osteosynthesen wie beispielsweise Platten, Nägel oder Schrauben, die im Körper eingepflanzt werden.

Unter einem "Metallimplantat" wird hier ein Kobalt-Chrom-Molybdän-basiertes, Edelstahlbasiertes oder ein Titanimplantat, insbesondere als Hüft-/Knieprothese oder Zahnimplantat, verstanden.

Der Begriff "Arthroplastik" (auch "Alloarthroplastik") wird hier insbesondere in Bezug auf den Einsatz von Gelenkendoprothesen, d.h. den Ersatz einer oder mehrerer Gelenkflächen durch allogenes, d.h. körperfremdes, nicht biologisches Material verwendet.

Der Begriff "Polymorphismus" bezeichnet Sequenzvariationen in den Genen einer Population. Verschiedene Individuuen einer Population oder auch verschiedene Zellen eines Individuums können bedingt durch solche Sequenzvariationen unterschiedliche Varianten eines bestimmten Gens am gleichen Genort (Locus) aufweisen, die auch als "Allele" bezeichnet werden. Der Begriff umfasst beispielsweise Variationen in einzelnen Nukleotiden (SNVs oder SNPs) oder das Auftreten einer verschiedenen Anzahl von Sequenzwiederholungen (z.B. Minisatellitensequenzen, VNTRs).

Unter dem Begriff "Einzelnukleotidvariation rs1143627" (ggfs. auch als IL-1B-31 bezeichnet) wird hier eine ein einzelnes Nukleotid an Position -31 (bezogen auf das Startcodon) in der Promotorregion des für Interleukin 1 beta (IL-1B, IL-1β) kodierenden Gens betreffende Variation bezeichnet, wobei an der Position ein C oder ein T stehen kann (z.B. NG_008851.1:g.4970C>T gemäß HGVS-Nomenklatur-Empfehlung, s. [11]). Eine Sequenz der betreffenden Region mit der Einzelnukleotidvariation (SNV, single nucleotide variation) ist im Folgenden wiedergegeben:
AGCCTCCTACTTCTGCTTTTGAAAGC[C/T]ATAAAAACAGCGAGGGAGAACTGG (SEQ ID NO: 1)

Die in Frage kommenden Nukleotid-Varianten (hier C oder T) sind in eckigen Klammern angegeben. Anstelle des Ausdrucks "Einzelnukleotidvariation" oder SNV wird gegebenenfalls synonym auch der Begriff "Einzelnukleotidpolymorphismus" oder SNP (single nucleotide polymorphism) verwendet.

Unter dem Begriff "VNTR-Polymorphismus rs2234663" (ggfs. auch als "Intron 2 VNTR rs2234663" oder "Intron 2 VNTR bezeichnet) wird ein Polymorphismus verstanden, bei dem eine variable Anzahl (2-6) von tandemartig hintereinanderliegenden Wiederholungseinheiten (VNTR, variable number tandem repeat) mit der Sequenz 
ATCCTGGGGAAAGTGAGGGAAATATGGACATCACATGGAACAACATCCAGGAGAC
TCAGGCCTCTAGGAGTAACTGGGTAGTGTGC (SEQ ID NO: 2)
 in dem Gen vorhanden ist, das für den II,1-Rezeptorantagonisten (II,1-AN oder II,1-RN) kodiert. Eine Sequenz mit vier Wiederholungseinheiten ist beispielsweise in der LRG-Sequenz (LRG = Locus Reference Genomic sequence) LRG_188 wiedergegeben (s. http://ftp.ebi.ac.uk/pub/databases/lrgex/LRG_188.xml; s. auch NCBI Referenzsequenz mit der Zugriffsnummer NG_021240.1). Gemäß HGVS-Nomenklatur-Empfehlung (s. [11]) wäre der VNTR-Polymorphismus mit fünf Wiederholungseinheiten mit g.17637_17722[5] zu bezeichnen.

Unter dem Begriff "atopische Erkrankung" wird eine Erkrankung verstanden, die auf einer genetisch bestimmten Bereitschaft beruht, auf aerogenen, gastrointestinalen oder kutanen Kontakt mit natürlichen oder künstlichen Umweltstoffen mit einer allergischen Reaktion des Soforttyps (Typ-I-Allergie), d.h. mit einer gesteigerten IgE-Bildung zu reagieren. Bei atopischen Erkrankungen sind häufig die Grenzflächen der respiratorischen und gastrointestinalen Schleimhäute sowie die Haut involviert. Beispiele für eine atopische Erkrankung sind allergisches Asthma bronchiale, allergische Rhinitis (allergische Rhinokonjunktivitis) und atopische Dermatitis (atopisches Ekzem, Neurodermitis).

Bevorzugt wird bei dem erfindungsgemäßen Verfahren der VNTR-Polymorphismus rs2234663 mit der Wiederholungseinheit 
ATCCTGGGGAAAGTGAGGGAAATATGGACATCACATGGAACAACATCCAGGAGAC
TCAGGCCTCTAGGAGTAACTGGGTAGTGTGC, SEQ ID NO: 2,
 im Gen kodierend für den II,1-Rezeptorantagonisten, II,1-RN, untersucht und festgestellt, ob die Wiederholungseinheit fünfmal vorhanden ist.

Besonders bevorzugt wird bei dem erfindungsgemäßen Verfahren zusätzlich ermittelt, ob bei der Testperson eine atopische Erkrankung vorliegt. Beispiele für atopische Erkrankungen sind Asthma bronchiale, allergische Rhinokonjunktivitis und atopische Dermatitis. Insbesondere bei einer Kombination aus dem Vorliegen eines VNTR-Polymorphismus rs2234663 mit fünf Wiederholungseinheiten und einer atopischen Erkrankung kann auf ein erhöhtes Risiko geschlossen werden, dass bei der betroffenen Person bei Implantation eines Metallimplantates Komplikationen, insbesondere verstärkte Entzündungsreaktionen, auftreten werden.

Einen weiteren Aspekt betrifftein Kit zur Durchführung des erfindungsgemäßen Verfahrens, das nicht Teil der Erfindung ist.

Das Kit umfasst die in Tabelle 1 angegebenen Primersequenzen:

**Tabelle 1**

| Polymorphismus | Primersequenzen | SEQ ID NO | Annealing-Temperatur |
|---|---|---|---|
| IL-1B-31 | TCTTTTCCCCTTTCCTTTAACT | 10 | 52°C |
| (rs1143627) | GAGAGACTCCCTTAGCACCTAGT | 11 | |
| IL-1RN | | | |
| Intron 2 VNTR | CCCCTCAGCAACACTCC | 12 | 65°C |
| (rs2234663) | GGTCAGAAGGGCAGAGA | 13 | |

Die Erfindung wird im Folgenden anhand von Ausführungsbeispielen rein zu Veranschaulichungszwecken näher beschrieben.

Es wurde eine Studie durchgeführt, an der unter Einwilligung nach erfolgter Aufklärung 195 Patienten mit Totalendoprothesen teilnahmen. Die Studie wurde durch die lokale Ethikkommission bewilligt und unter der Studien-ID 230-12 unter ClinicalTrials.gov registriert. 102 Patienten (72 weiblich, 30 männlich, 41-81 Jahre) wiesen Arthroplastiken mit Komplikationen auf. Die Kontrollgruppe bestand aus 93 Patienten (70 weiblich, 23 männlich, 18-96 Jahre) mit normal funktionierenden Arthroplastiken.

### Fragebogen

Es wurde eine fragebogenunterstützte Anamnese erhoben, die Informationen über Rauchen, Medikamenteneinnahme, Implantattyp, Metallallergie-Vorgeschichte und die Vorgeschichte atopischer Erkrankungen wie allergische Rhinitis, atopisches Ekzem oder Asthma enthielt. Zusätzlich wurde der orthopädische WOMAC-Fragebogen (Western Ontario and McMaster Universities Osteoarthritis Index, WOMAC) verwendet, um die Selbsteinschätzung des Patienten hinsichtlich des Verhaltens des künstlichen Gelenks bezüglich Schmerzen, Steifheit und Funktionseinschränkung zu quantifizieren [5].

### Allergene und Patch-Tests

Alle Patienten wurden im Patch-Test allergologisch getestet. Dafür wurden Almirall-Hermal-Patch-Testpräparate und Finn-Kammern auf Scanpor (Almirall Hermal, Reinbek, Deutschland) verwendet. Die Substanzen wurden am Tag 0 auf den oberen Rücken aufgebracht. Die Standardserie mit 29 Allergenen, eine routinemäßige Ergänzungsserie und eine Knochenzementserie (wenn die jeweiligen Patienten eine zementierte Endoprothese hatten) nach den Richtlinien der Deutschen Kontaktdermatitis-Forschungsgruppe (DKG) wurde getestet [6]. Die Messungen wurden von Ärzten der Allergieabteilung am 2., 3. und 6. Tag durchgeführt. Die Reaktionen, die hier als als positiv beschrieben sind, waren Reaktionen, die als +, ++ und +++ klassifiziert wurden.

### Lymphozytentransformationstest (LTT)

Ein Lymphozytentransformationstest (LTT) wurde gemäß Summer et al. [7] durchgeführt. Kurz zusammengefasst, wurden mononukleäre Zellen des peripheren Blutes (PBMC) durch Dichtezentrifugation isoliert und vier Tage lang in vier Parallelansätzen mit verschiedenen Konzentrationen von NiSO₄, CoCl₂ oder CrCl₃ stimuliert. Kontrollstimuli waren das T-Zell-Mitogen Phytohämagglutinin (PHA, Biochrom, Berlin, Deutschland) 2,4 µg/ml, Tetanustoxoid als Kontroll-Recall-Antigen (TT, Chiron Behring, Berlin, Deutschland) 5 µg/ml. Nach 5 Tagen wurden die PBMC mit ³H-Thymidin gepulst und die Proliferation wurde nach Inkubation über Nacht durch Messung der aufgenommenen Radioaktivität ermittelt. Die Ergebnisse sind als Stimulationsindex (SI) angegeben, was das Verhältnis von aufgenommener Radioaktivität bei stimulierten gegenüber nicht-stimulierten Kontrollkulturen bedeutet. Ein SI ≥3 wurde als eine positive Reaktion angesehen.

### Polymorphismus-Analyse

DNA wurde aus dem peripheren Blut des Patienten mit dem "peqGOLD Blood DNA Mini Kit" (Peqlab, Erlangen, Deutschland) gemäß dem Protokoll des Herstellers isoliert. Die erhaltene DNA wurde mit destilliertem H₂0 auf eine Endkonzentration von 40 ng/µl verdünnt. Die drei Polymorphismen IL-1B-3954 (rs1143634), IL-1B-31 (rs1143627), IL1-B-511 (rs16944) und die Minisatellitensequenz IL-1 RN Intron 2 VNTR (rs2234663) wurden analysiert durch PCR, Verdau mit den Restriktionsenzymen Taql, Alu1 oder Ava1, gefolgt von Gelelektrophorese. Die DNA-Sequenzen der Polymorphismen sind in Tabelle 1 angegeben.

**Tabelle 2: Untersuchte Polymorphismen und die entsprechenden DNA-Sequenzen**

| Polymorphismus | DNA-Sequenz |
|---|---|
| IL-1 B-3954 (rs1143634) | CTCCACATTTCAGAACCTATCTTCTT[C/T]GACACATGGGATA ACGAGGCTTATG (SEQ ID NO:3) |
| IL-1 B-511 (rs 16944) | CTACCTTGGGTGCTGTTCTCTGCCTC[A/G]GGAGCTCTCTGTC AATTGCAGGAGC (SEQ ID NO: 4) |
| IL-1B-31 (rs1143627) | AGCCTCCTACTTCTGCTTTTGAAAGC[C/T]ATAAAAACAGCG AGGGAGAACTGG (SEQ ID NO: 1) |
| IL-1RN Intron 2 VNTR (rs2234663) | ACTCCTATTGACCTGGAGCACAGGT[(ATCCTGGGGAAAGTG AGGGAAATATGGACATCACATGGAACAACATCCAGGAGA CTCAGGCCTCTAGGAGTAACTGGGTAGTGTGC)2/3/4/5/6]TT GGTTTA (SEQ ID NO: 5–9) |

Die Polymorphismen mit ihrem jeweiligen Restriktionsenzym und die resultierenden Fragmente sind in Tabelle 2 angegeben.

**Tabelle 2: Die analysierten Polymorphismen mit den jeweiligen Restriktionsenzymen und den resultierenden DNA-Fragmentlängen**

| Polymorphismus | Restriktionsenzym | Fragmentlänge (bp) |
|---|---|---|
| IL-1B-3954 (rs1143634) | Taq I | T: 250 bp |
| | | C: 136 bp + 114 bp |
| IL-1B-31 (rs1143627) | Alu 1 | C: 281 bp |
| | | T: 184+97bp |
| IL-1 B-511 (rs16944) | Ava 1 | A 304 bp |
| | | G 189 bp + 115 bp |
| IL-1RN | Ø | Allel 1: 412 bp (4 repeats) |
| Intron 2 VNTR (rs2234663) | | Allel 2: 240 bp (2 repeats) |
| | | Allel 3: 498 bp (5 repeats) |
| | | Allel 4: 326 bp (3 repeats) |
| | | Allel 5: 584 bp (6 repeats) |

### Statistik

Die statistische Auswertung erfolgte in Zusammenarbeit mit dem Institut für Statistik der Ludwig-Maximilians-Universität München anhand von Kreuztabellen mit exakten Fisher-Tests mit einem Allelmodell sowie einer Risikoanalyse (Odds Ratio) nach mehreren Autoren [8-10].

### Ergebnisse

Patienten mit Arthroplastik-Komplikationen zeigten verschiedene Symptome wie zum Beispiel Schmerzen (84,1%), gefolgt von einer Verringerung der Bewegungsfreiheit (78,4%), Schwellungen (71,6%) und anderen. Dies spiegelt sich auch im WOMAC-Wert wider, wo 100 Punkte perfekte Funktionalität bedeuten. Hier bestätigten die Arthorplastik-Kontroll-Patienten die gute Leistung der Endoprothetik mit einem hohen Mittelwert von 82,13 ± 18,11. Auf der anderen Seite zeigten Patienten mit Arthroplastik-Komplikationen einen niedrigen mittleren WOMAC-Wert von 42,06 ± 10,12. In der Gruppe mit Arthroplastik-Komplikationen war die Rate an atopischen Erkrankungen (24,5% vs. 16,1%) höher, wobei Heuschnupfen die größte Fraktion darstellte (18,7% vs. 11,8%). Es gab auch eine höhere Anzahl von Patienten mit anamnestischer Metallüberempfindlichkeit in der Gruppe mit Arthroplastik-Komplikationen (24,05% vs 9,7%). Die Ergebnisse des positiven Metall-Patchtests waren wie folgt: Nickel 19,6% vs. 9,7%, Kobalt 6,9% vs. 3,2%, Chrom 2,0% vs. 1,1%. Eine größere Anzahl von Patienten war positiv im LTT. Hier hatten 28,4% der Patienten mit Arthroplastik-Komplikationen einen positiven LTT gegenüber Nickel, 2,9% gegenüber Kobalt und 2,9% gegenüber Chrom. Weniger Patienten der Kontrollgruppe hatten einen positiven LTT mit 19,4% gegenüber Nickel, 1,1% gegenüber Kobalt und keiner gegenüber Chrom. Die Ergebnisse von Fragebogen, Patch-Test und LTT sind in Tabelle 3 aufgeführt.

**Tabelle 3: Ergebnisse von Fragebogen, Patch-Test und LTT in den Patientengruppen. MW = Mittelwert, SD = Standardabweichung**

| | Arthroplastik-Patienten mit Beschwerden (n=102) | | Arthroplastik-Kontroll-Patienten (n=93) | |
|---|---|---|---|---|
| Beschwerden: | 96 | 94,1% | 0 | 0% |
| Schmerzen | 15 | 14,7% | 0 | 0% |
| Ekzem | 7 | 6,9% | 0 | 0% |
| Erguss | 31 | 30,4% | 0 | 0% |
| Schwellung | 73 | 71,6% | 0 | 0% |
| Lockerung | 20 | 19,6% | 0 | 0% |
| Bewegungseinschränkung | 80 | 78,4% | 0 | 0% |
| WOMAC-Wert (MW ± SD | 42,06 | ±10,12% | 92,13 | ±18,11% |
| | | | | |
| Rauchen: | | | | |
| Früher | 19 | 18,6% | 16 | 16,7% |
| Aktuell | 7 | 6,9% | 4 | 3,8% |
| Insgesamt | 26 | 25,5% | 20 | 20,5% |
| | | | | |
| Atopie: | | | | |
| Atopie (insgesam) | 25 | 24,5% | 15 | 16,1% |
| Heuschnupfen | 19 | 18,7% | 11 | 11,8% |
| Asthma | 13 | 12,7 | 5 | 5,4% |
| Atopisches Ekzem | 0 | 0,0% | 0 | 0,0% |
| Metall-Überempfindlichkeit (anamnestisch) | 25 | 24,5% | 9 | 9,7% |
| | | | | |
| Patch-Test: | | | | |
| Nickel-positiv | 20 | 19,6% | 9 | 9,7% |
| Kobalt-positiv | 7 | 6,9% | 3 | 3,2% |
| Chrom-positiv | 2 | 2,0% | 1 | 1,1% |
| Gentamicin-positiv | 9 | 8,8% | 0 | 0,0% |
| Benzoylperoxid-positiv | 8 | 7,8% | 2 | 2,2% |
| HEMA-positiv | 2 | 2,0% | 0 | 0,0% |
| N,N-Dimethyl-p-toluidin-positiv | 2 | 2,0% | 0 | 0,0% |
| | | | | |
| LTT: | | | | |
| Nickel-positiv | 29 | 28,4% | 18 | 19,4% |
| Kobalt-positiv | 3 | 2,9% | 1 | 1,1% |
| Chrom-positiv | 3 | 2,9% | 0 | 0,0% |

Es gab keine Unterschiede in den Allelhäufigkeiten der zwei verschiedenen Allele C oder T des II,-1B-Polymorphismus 3954. Auch die Subgruppenanalyse bei Patienten mit oder ohne Atopie, positivem oder negativem Patch-Test oder LTT, zeigte keine relevanten Unterschiede. Es gab einen leichten Trend für eine höhere Häufigkeit des C-Allels in der Kontrollgruppe, insbesondere bei den Patienten ohne Atopie, ohne positiven Patch-Test oder positive LTT-Reaktion. Die Ergebnisse für den IL1B-Polymorphismus 3954 sind in Tabelle 4 aufgeführt.

**Tabelle 4: Allelfrequenzen des IL-1 B-3954-Polymorphismus auch einschließlich Untergruppen-Analyse. Es sind Ergebnisse von Fishers exaktem Test und Odds-Ratio Fisher (+ 95% Konfidenzintervall, KI) angegeben.**

| Allele IL-1B 3954 | | Arthroplastik-Patienten mit Beschwerden (n= 102) | | Arthroplastik-Kontroll-Patienten (n=93) | p (Fishers exakter Test) | | Odds-Ratio [95% KI] |
|---|---|---|---|---|---|---|---|
| | | Allelhäufigkeiten (n=204) | [%] | Allelhäufigkeiten (n=186) | [%] | | |
| Insgesamt | C | 184 | 90,2 | 174 | 95,6 | 0,050 | 0,423 [0,182-0,985] |
| | T | 86 | 42,2 | 72 | 39,6 | 0,682 | 1,113 [0,741-1,672] |
| Mit | C | 46 | 92,0 | 28 | 93,3 | 1,000 | 0,821 [0,141-4,780] |
| Atopie | T | 22 | 44,0 | 18 | 60,0 | 0,254 | 0,524 [0,209-1,314] |
| keine | C | 134 | 89,3 | 146 | 96,1 | 0,028 | 0,344 [0,131-0,905] |
| Atopie | T | 62 | 41,3 | 54 | 35,5 | 0,344 | 1,279 [0,803-2,035] |
| Patch-Test | C | 46 | 95,8 | 24 | 92,3 | 0,609 | 1,917 [0,254-14,465] |
| pos. | T | 18 | 37,5 | 12 | 46,2 | 0,620 | 0,700 [0,266-1,842] |
| Patch-Test | C | 138 | 88,5 | 150 | 96,2 | 0,018 | 0,307 [0,118-0,985] |
| neg. | T | 68 | 43,6 | 60 | 38,5 | 0,420 | 1,236 [0,103-2,748] |
| LTT | C | 52 | 89,7 | 32 | 94,1 | 0,705 | 0,542 [0,103-2,748] |
| pos. | T | 30 | 51,7 | 18 | 52,9 | 1,000 | 0,925 [0,408-2,223] |
| LTT | C | 94 | 87,0 | 106 | 94,6 | 0,061 | 0,380 [0,140-1,029] |
| neg. | T | 46 | 42,6 | 38 | 33,9 | 0,212 | 1,445 [0,837-2,495] |

Der IL-1B-31-Polymorphismus mit dem Allel T hatte höhere Frequenzen in der Kontrollgruppe (84,3% vs 48%). Dies kann einen "schützenden" Genotyp widerspiegeln, der eine immunologische Antwort auf das Implantat reduzieren kann. Dieser Unterschied in den T-Allelfrequenzen war auch in der Subgruppenanalyse vorhanden. Die Ergebnisse der IL-1B-31-Allelfrequenzen sind in Tabelle 5 aufgeführt.

**Tabelle 5: Allelfrequenzen des IL-1B-31-Polymorphismus einschließlich der Subgruppenanalyse. Die Ergebnisse von Fishers exaktem Test und Odds-Ratio (+ 95%-Konfidenzintervall, KI) sind angegeben.**

| Allele IL-1B -31 | | | | Arthroplastik-Patienten mit Beschwerden | Arthroplastik-Kontroll-Patienten | p (Fishers exakter | Odds-Ratio [95% KI] |
|---|---|---|---|---|---|---|---|
| | | (n=102) | | (n=93) | | Test) | |
| | | Allelhäufigkeiten (n=204) | [%] | Allelhäufigkeiten (n=186) | [%] | | |
| Insgesamt | T | 98 | 48,0 | 150 | 84,3 | 0,00000052 | 0,173 [0,106-0,281] |
| | C | 156 | 76,5 | 112 | 62,9 | 0,005 | 1,915 [1,228-2,986] |
| Mit | T | 18 | 36,0 | 26 | 86,7 | 0,00008 | 0,087 [0,028-0,288] |
| Atopie | C | 42 | 84,0 | 18 | 60,0 | 0,031 | 3,500 [1,223-10,015] |
| keine | T | 76 | 50,7 | 124 | 83,8 | 0,0000092 | 0,199 [0,116-0,342] |
| Atopie | C | 110 | 73,3 | 94 | 63,5 | 0,081 | 1,580 [0,965-2,586] |
| Patch-Test | T | 24 | 50,0 | 22 | 91,7 | 0,001 | 0,091 [0,019-0,430] |
| pos. | C | 38 | 79,2 | 14 | 58,3 | 0,093 | 2,714 [0,932-7,909] |
| Patch-Test | T | 74 | 47,4 | 128 | 83,1 | 0,0000003 | 0,183 [0,108-0,310] |
| neg. | C | 118 | 75,6 | 98 | 63,6 | 0,026 | 1,774 [1,086-2,900] |
| LTT | T | 24 | 41,4 | 30 | 88,2 | 0,000008 | 0,094 [0,029-0,302] |
| pos. | C | 44 | 75,9 | 16 | 47,1 | 0,007 | 3,536 [1,433-8,722] |
| LTT | T | 50 | 46,3 | 88 | 81,5 | 0,000001 | 0,196 [0,106-0,363] |
| neg. | C | 84 | 77,8 | 76 | 70,4 | 0,277 | 1,474 [0,798-2,722] |

Es gab keine relevanten Unterschiede in den Allelfrequenzen des II,-1B-511-Polymorphismus. Es gab einen Trend für eine höhere Häufigkeit des C-Allels dieses Polymorphismus in der Kontrollgruppe, aber dies war nicht hoch genug statistisch signifikant. Die Ergebnisse für den IL-1 B-511-Polymorphismus sind in Tabelle 6 aufgeführt.

**Tabelle 6: Allelfrequenzen des II,-1B-511-Polymorphismus einschließlich der Subgruppenanalyse. Die Ergebnisse von Fishers exaktem Test und Odds-Ratio (+ 95%-Konfidenzintervall, KI) sind angegeben.**

| Allele IL-1B 511 | | | | Arthroplastik-Patienten mit Beschwerden | Arthroplastik-Kontroll-Patienten | p (Fishers exakter | Odds-Ratio [95% KI] |
|---|---|---|---|---|---|---|---|
| | | (n=102) | | (n=93) | | Test) | |
| | | Allelhäufigkeiten (n=204) | [%] | Allelhäufigkeiten (n=186) | [%] | | |
| Insgesamt | C | 150 | 73,5 | 158 | 87,8 | 0,0005 | 0,387 [0,225-0,666] |
| | T | 130 | 63,7 | 106 | 58,9 | 0,346 | 1,226 [0,812-1,851] |
| Mit | C | 30 | 60,0 | 22 | 73,3 | 0,333 | 0,545 [0,203-1,464] |
| Atopie | T | 36 | 72,0 | 18 | 60,0 | 0,327 | 1,714 [0,659-1,675] |
| keine | C | 116 | 77,3 | 136 | 90,7 | 0,003 | 0,351 [0,180-0,686] |
| Atopie | T | 90 | 60,0 | 88 | 58,7 | 0,906 | 1,057 [0,667-1,675] |
| Patch-Test | C | 34 | 70,8 | 24 | 92,3 | 0,040 | 0,202 [0,042-0,974] |
| pos. | T | 30 | 62,5 | 16 | 61,5 | 1,000 | 1,042 [0,390-2,783] |
| Patch-Test | C | 116 | 74,4 | 134 | 87,8 | 0,006 | 0,433 [0,240-0,782] |
| neg. | T | 100 | 64,1 | 90 | 58,4 | 0,351 | 1,270 [0,803-2,007] |
| LTT | C | 40 | 69,0 | 30 | 88,2 | 0,044 | 0,296 [0,091-0,966] |
| pos. | T | 38 | 65,5 | 20 | 58,8 | 0,655 | 1,330 [0,556-3,180] |
| LTT | C | 82 | 75,9 | 98 | 87,5 | 0,035 | 0,451 [0,221-0,919] |
| neg. | T | 70 | 64,8 | 72 | 64,3 | 1,000 | 1,023 [0,589-1,778] |

Beim II,-1B-RN-Intron-2-VNTR gab es ein viel höheres Risiko für arthroplastische Beschwerden, die mit dem Allel 3 (498 bp, 5 Wiederholungseinheiten) korrelierten. Interessanterweise war dieses Risiko erhöht, wenn die Patienten zusätzlich eine atopische Erkrankung hatten. Dieses Risiko korrelierte nicht mit der Patch-Test-Reaktion oder der LTT-Reaktivität. Die Ergebnisse für das IL-1B-RN Intron 2 VNTR sind in Tabelle 7 aufgeführt.

**Tabelle 7: Allelfrequenzen des IL-1B-RN-VNTR-Polymorphismus einschließlich der Subgruppenanalyse. Die Ergebnisse von Fishers exaktem Test und Odds-Ratio (+ 95%-Konfidenzintervall, KI) sind angegeben.**

| Allele IL-1B | | | | Arthroplastik-Patienten mit | Arthroplastik-Kontroll- | p (Fishers | Odds-Ratio [95% KI] |
|---|---|---|---|---|---|---|---|
| RN VNTR | | Beschwerden (n=102) | | Patienten (n=93) | | exakter Test) | |
| | Allelhäufigkeiten (n=204) | | [%] | Allelhäufigkeiten (n=186) | [%] | | |
| Insgesamt | 240bp | 48 | 27,6 | 44 | 28,2 | 0,903 | 0,970 [0,599-1,570] |
| | 326bp | 6 | 3,4 | 6 | 3,8 | 1,000 | 0,905 [0,289-2,865] |
| | 412bp | 128 | 72,7 | 136 | 86,1 | 0,030 | 0,431 [0,247-0,755] |
| | 498bp | 86 | 49,4 | 36 | 22,8 | 0,0000475 | 3,312 [2,058-5,331] |
| | 595bp | 6 | 3,4 | 0 | 0,0 | 0,031 | 0515 [0,464-0,573] |
| Mit | 240bp | 8 | 19,0 | 10 | 33,3 | 0,182 | 0,471 [0,160-1,138] |
| Atopie | 326 bp | 0 | 0,0 | 2 | 6,7 | 0,170 | 0,400 [0,300-0,533] |
| | 412bp | 30 | 71,4 | 28 | 93,3 | 0,032 | 0,179 [0,037-0,870] |
| | 498bp | 36 | 85,7 | 4 | 13,3 | 0,00000001 | 39,000 [9,990-152,257] |
| | 595bp | 2 | 4,8 | 0 | 0,0 | 0,507 | 0,571 [0,467-0,700] |
| keine | 240bp | 40 | 31,3 | 34 | 27,0 | 0,491 | 1,230 [0,715-2,116] |
| Atopie | 326bp | 6 | 4,7 | 4 | 3,1 | 0,749 | 1,525 [0,420-5,536] |
| | 412bp | 94 | 72,3 | 108 | 84,4 | 0,023 | 0,484 [0,262-0,892] |
| | 498bp | 50 | 39,1 | 32 | 25,0 | 0,022 | 1,923 [1,126-3,283] |
| | 595bp | 4 | 3,1 | 0 | 0,0 | 0,122 | 0,492 [0,434-0,558] |
| Patch-Test | 240bp | 4 | 9,1 | 2 | 11,1 | 1,000 | 0,800 [0,133-4,809] |
| pos. | 326bp | 0 | 0 | 0 | 0 | n.b. | n.b. |
| | 412bp | 34 | 77,3 | 18 | 90,0 | 0,312 | 0,378 [0,075-1,913] |
| | 498bp | 26 | 59,1 | 4 | 20,0 | 0,006 | 5,778 [1,656-20.159] |
| | 595bp | 2 | 4,5 | 0 | 0 | 1,000 | 0,677 [0,571-0,804] |
| Patch-Test | 240bp | 44 | 33,8 | 42 | 30,4 | 0,601 | 1,169 [0,700-1,954] |
| neg. | 326bp | 6 | 4,6 | 6 | 4,3 | 1,000 | 1,065 [0,334-3,388] |
| | 412bp | 94 | 71,2 | 118 | 85,4 | 0,005 | 0,419 [0,229-0,768] |
| | 498bp | 60 | 46,2 | 32 | 23,2 | 0,0001 | 2,839 [1,680-4,798] |
| | 595bp | 4 | 3,1 | 0 | 0 | 0,054 | 0,477 [0,421-0,541] |
| LTT | 240bp | 8 | 16,7 | 4 | 14,3 | 1,000 | 1,200 [0,326-4,414] |
| pos. | 326bp | 4 | 8,3 | 0 | 0 | 0,290 | 0,611 [0,508-0,735] |
| | 412bp | 38 | 76,0 | 26 | 92,2 | 0,073 | 0,244 [0,050-1,180] |
| | 498bp | 30 | 62,5 | 6 | 21,4 | 0,001 | 6,111 [2,085-17,911] |
| | 595bp | 0 | 0 | 0 | 0 | n.b. | n.b. |
| LTT | 240bp | 30 | 31,3 | 32 | 32,0 | 1,000 | 0,966 [0,529-1,764] |
| neg. | 326bp | 2 | 2,1 | 6 | 5,9 | 0,281 | 0,340 [0,067-1,729] |
| | 412bp | 72 | 75,0 | 84 | 82,4 | 0,227 | 0,643 [0,323-1,278] |
| | 498bp | 44 | 45,8 | 24 | 23,5 | 0,001 | 2,750 [1,496-5,055] |
| | 595bp | 2 | 2,1 | 0 | 0 | 0,234 | 0,480 [0,415-0,555] |

### Referenzliste

1. Sharkey PF et al. Why are total knee arthroplasties failing today. J Arthroplasty 2017; 29:1774-1778
2. Thomsen M et al. Adverse Reaktionen gegenüber orthopädisch-chirurgischen Metallimplantaten nach Kniegelenkersatz. Hautarzt 2016;67:347-351
3. Granchi D et al. Sensitivity to implant materials in patients undergoing total hip replacement. J Biomed Mater Res B Appl Biomater 2006;77(2):257-64
4. Thomas P, Summer B. Diagnosis and management of patients with allergy to metal implants. Exp Rev Clin Immunol. 2015;11(4):501-509
5. Stucki G et al. Evaluation einer deutschen Version des WOMAC (Western Ontario und McMaster Universities) Arthroseindex. Z Rheumatol 1996;55(1):40-49
6. Schnuch A et al. Durchführung des Epikutantests mit Kontaktallergenen. Leitlinie der Deutschen Dermatologischen Gesellschaft und der Deutschen Gesellschaft für Allergie und klinische Immunologie. JDDG 2008;6(9):770-775
7. Summer B et al. Nickel (Ni) allergic patients with complications to Ni containing joint replacement show preferential IL-17 type reactivity to Ni. Contact Dermatitis 2010;63:15-22
8. Colhoun HM et al. Problems of reporting genetic associations with complex outcomes. Lancet. 2003;361(9360):865-72
9. Yang H et al. Association of interleukin gene polymorphisms with the risk of coronary artery disease. Genetics and molecular research : GMR. 2015;14(4):12489-96.
10. Lopez-Mejias R et al. Interleukin 1 beta (IL1ss) rs16944 genetic variant as a genetic marker of severe renal manifestations and renal sequelae in Henoch-Schonlein purpura. Clinical and experimental rheumatology. 2016;34(3 Suppl 97):S84-8.
11. den Dünnen et al., HGVS Recommendations for the Description of Sequence Variants: 2016 Update, HUMAN MUTATION, Vol. 37, No. 6, 564-569, 2016, doi10.1002/humu.22981.

## Patentansprüche

1. Verfahren zur Prognostizierung eines erhöhten Risikos für eine erhöhte Unverträglichkeitsneigung gegenüber Metallimplantaten bei einer Testperson, wobei in einer peripheren Blutprobe mit genetischem Material der Testperson:
a) eine Einzelnukleotidvariation rs1143627 AGCCTCCTACTTCTGCTTTTGAAAGC[C/T]ATAAAAACAGCGAGGGAGAACTGG, SEQ ID NO: 1, im Gen kodierend für Interleukin 1 beta, untersucht und festgestellt wird, dass ein C an der Position der Einzelnukleotidvariation vorhanden ist und/oder
b) ein VNTR-Polymorphismus rs2234663 mit der Wiederholungseinheit 
ATCCTGGGGAAAGTGAGGGAAATATGGACATCACATGGAACAACATCCAGGAGAC
TCAGGCCTCTAGGAGTAACTGGGTAGTGTGC, SEQ ID NO: 2,
 im Gen kodierend für den II,1-Rezeptorantagonisten, II,1-RN, untersucht und festgestellt wird, dass die Wiederholungseinheit fünfmal vorhanden ist, und wobei es sich bei den Metallimplantaten um Gelenkendoprothesen handelt.

2. Verfahren nach Anspruch 1, wobei es sich bei der erhöhten Unverträglichkeitsneigung um eine erhöhte Entzündungsneigung handelt.

3. Verfahren nach Anspruch 1 oder 2, wobei zusätzlich das mögliche Vorliegen einer atopischen Erkrankung ermittelt wird.

4. Verfahren nach Anspruch 3, wobei das mögliche Vorliegen einer atopischen Erkrankung ermittelt wird, die ausgewählt ist aus allergischem Asthma bronchiale, allergischer Rhinitis und atopischer Dermatitis.

## Claims

1. Method for predicting an increased risk of increased incompatibility propensity with respect to metallic implants in a test person, wherein, in a peripheral blood sample with genetic material from the test person:
a) a SNV rs1143627 AGCCTCCTACTTCTGCTTTTGAAAGC[C/T]ATAAAAACAGCGAGGGAGAACTGG, SEQ ID NO: 1, in the gene encoding interleukin 1 beta, is analysed and it is determined that a C is present at the position of the SNV, and/or
b) a VNTR polymorphism rs2234663 with the repeating unit 
ATCCTGGGGAAAGTGAGGGAAATATGGACATCACATGGAACAACATCCAGGAGAC
TCAGGCCTCTAGGAGTAACTGGGTAGTGTGC, SEQ ID NO: 2,
in the gene encoding the IL1 receptor antagonist, IL1-RN, is analysed and it is determined that the repeating unit is present five times,
and wherein the metal implants are joint endoprostheses.

2. Method according to claim 1, wherein the increased incompatibility propensity is an increased inflammation propensity.

3. Method according to claim 1 or 2, wherein the possible presence of an atopic disease is additionally determined.

4. Method according to claim 3, wherein the possible presence of an atopic disease is determined, which is selected from allergic bronchial asthma, allergic rhinitis and atopic dermatitis.

## Revendications

1. Procédé pour le pronostic d'un risque élevé d'intolérance accrue à des implants métalliques chez une personne test, dans lequel, dans un échantillon sanguin périphérique avec du matériel génétique de la personne test :
a) un SNV rs1143627 AGCCTCCTACTTCTGCTTTTGAAAGC[C/T]ATAAAAACAGCGAGGGAGAA CTGG, SEQ ID NO: 1, dans le gène codant pour l'interleukine 1 bêta, est analysé et identifié pour déterminer qu'un C est présent à la position du SNV, et/ou
b) un polymorphisme de type VNTR rs2234663 avec le motif de répétition 
ATCCTGGGGAAAGTGAGGGAAATATGGACATCACATGGAACAACATCCA
GGAGAC TCAGGCCTCTAGGAGTAACTGGGTAGTGTGC, SEQ ID NO: 2,
dans le gène codant pour l'antagoniste au récepteur de l'IL1, IL1-RN, est analysé et identifié pour déterminer que le motif de répétition est présent cinq fois,
et dans lequel les implants métalliques sont des endoprothèses d'articulation.

2. Procédé selon la revendication 1, dans lequel l'intolérance accrue est une propension accrue à provoquer une inflammation.

3. Procédé selon la revendication 1 ou 2, dans lequel la présence éventuelle d'une maladie atopique est déterminée en supplément.

4. Procédé selon la revendication 3, dans lequel la présence éventuelle d'une maladie atopique est déterminée, laquelle est choisie parmi un asthme bronchique allergique, une rhinite allergique et une dermatite atopique.
